# EUROPEAN PATENT APPLICATION

(11) **EP 3 862 438 A1**
(43) Date of publication of application: **11.08.2021**
(21) Application number: 19868474.8
(22) Date of filing: 04.10.2019
(51) Int. Cl.: C12Q 1/68, A61K 31/7105, A61K 45/00, A61K 48/00, A61P 13/12, A61P 43/00, C12N 15/11

(54) **ACUTE RENAL FAILURE-SPECIFIC BIOMARKER, ACUTE RENAL FAILURE DIAGNOSIS METHOD, ACUTE RENAL FAILURE TEST KIT, ANIMAL TREATMENT METHOD AND ACUTE RENAL FAILURE MEDICATION**

(30) Priority: 04.10.2018 JP 2018188746
(71) Applicant: Jichi Medical University, Tokyo 102-0093 (JP)
(72) Inventor: MORISHITA, Yoshiyuki, Saitama-shi, Saitama 330-8503 (JP)
(74) Representative: Grünecker Patent- und Rechtsanwälte PartG mbB
(86) International application number: PCT/JP2019/039227
(87) International publication number: WO 2020/071518

(57) **Abstract**

Provided is a biomarker capable of specifically diagnosing acute kidney injury. An acute kidney injury-specific biomarker is a miRNA that is primarily present in the blood. The miRNA is miRNA-5100. Furthermore, miRNA-5100 can be used as a method for diagnosing acute kidney injury. In this diagnosis, when the expression of miRNA-5100 is decreased in the blood, it is diagnosed as acute kidney injury. As an acute kidney injury test kit, it is also possible to include a reagent for measuring miRNA-5100.

## Description

### BACKGROUND

### [Technical field]

The present invention relates to a biomarker, a diagnostic method, a test kit, an animal treatment method, and a medicine, and in particular, an acute kidney injury-specific biomarker, an acute kidney injury diagnosis method, an acute kidney injury test kit, an animal treatment method, and an acute kidney injury medication.

### [Background technology]

Acute kidney injury (AKI) is an acute renal dysfunction that occurs after infection, drug intake, surgery, or the like, and is greatly involved in the prognosis of a patient's life.

Acute kidney injury (AKI) is a condition in which renal function rapidly declines in a short period of several hours to several days. Waste products cannot be excreted from urine, or the water overflows. It sometimes happens that dialysis may be required.

Conventionally, Kidney Disease: Improving Global Outcomes (KDIGO) has provided guidelines for diagnosis of acute kidney injury. In this KDIGO classification, acute renal failure is diagnosed on the basis of elevated serum creatinine levels and decreased urine output.

Specifically, (in the KDIGO Practice Guidelines for Acute Kidney Injury), the stage classification of AKI is defined by one of the following:
1. When serum creatinine level rises by 0.3 mg / dl or more within 48 hours.
2. When the serum creatinine level was known within 7 days before that, or When there is an increase of 1.5 times or more from the expected base value
3. When urine volume decreases to 0.5 ml / kg / hour over 6 hours.

However, this criterion often misses the timing of treatment interventions. Therefore, the development of biomarkers for acute kidney injury is desired.

By the way, in recent years, it has been reported that microRNA (miRNA , micro-RNA, miR) is involved in various pathological conditions and is effective as a biomarker for early diagnosis of diseases.

With reference to Patent Document 1, as an example of a conventional biomarker, miRNA indicating an index of a chronic diabetic condition is described.

### [Prior Art Document]

### [Patent Document]

[Patent Document 1] JP2013-514277A

### [Summary of Invention]

### [Problems to be Solved by the Invention]

However, the biomarker in Patent Document 1 has not been able to diagnose acute kidney injury.

The present invention has been made in view of such a situation, and an object of the present invention is to solve the above-mentioned problems.

### [Means for solving problems]

An acute kidney injury-specific biomarker of the present invention is an acute kidney injury-specific biomarker, which is miRNA-5100.

An acute kidney injury diagnosis method of the present invention is using the acute kidney injury-specific biomarker.

The acute kidney injury diagnosis method of the present invention is diagnosing as acute kidney injury when the expression of miRNA-5100 is decreased.

An acute kidney injury test kit of the present invention includes a reagent for measuring miRNA-5100.

An animal treatment method of the present invention is a treatment method for an animal other than a human and is regulating functional activity of miRNA-5100.

An acute kidney injury medication of the present invention includes a functional activity regulator of miRNA-5100.

The acute kidney injury medication of the present invention is, wherein the functional activity regulator includes the functional activity enhancer of miRNA-5100.

The medicament for acute kidney injury of the present invention is, wherein the functional activity enhancer is a miRNA mimic corresponding to the miRNA-5100.

### [Effect of the invention]

According to the present invention, the use of miRNA-5100 can provide a biomarker capable of specifically diagnosing acute kidney injury.

### [Simple explanation of drawings]

FIG. 1 is a conceptual diagram of an operation of an acute kidney injury model mouse according to Example 1 of the present invention.
FIG. 2A is a photograph showing results of microarray analysis of ischemia-reperfusion model according to Example 1 of the present invention.
FIG. 2B is a photograph showing results of microarray analysis of lipopolysaccharide (LPS) administration model according to Example 1 of the present invention.
FIG. 3 is a graph showing changes in the expression level of miRNA-5100 according to Example 1 of the present invention.
FIG. 4 is a graph showing the Receiver Operating Characteristic (ROC) curve of miRNA-5100 according to Example 1 of the present invention.
FIG. 5 is a conceptual diagram illustrating overexpression of miRNA-5100 in acute kidney injury mice according to Example 1 of the present invention.
FIG. 6 is a graph showing an actual expression level of miRNA-5100 at the time of overexpression according to Example 1 of the present invention.
FIG. 7 is a graph showing renal swelling during overexpression of miRNA-5100 according to Example 1 of the present invention.
FIG. 8A is a graph showing therapeutic effect (NGAL) of acute kidney injury at the time of overexpression of miRNA-5100 according to Example 1 of the present invention.
FIG. 8B is a graph showing therapeutic effect (KIM-1) of acute kidney injury at the time of overexpression of miRNA-5100 according to Example 1 of the present invention.
FIG. 8C is a graph showing therapeutic effect (L-FABP) of acute kidney injury at the time of overexpression of miRNA-5100 according to Example 1 of the present invention.
FIG. 8D is a graph showing therapeutic effect (IL-18) of acute kidney injury at the time of overexpression of miRNA-5100 according to Example 1 of the present invention.
FIG. 9 is a graph showing changes in expression of endoplasmic reticulum stress response gene during overexpression of miRNA-5100 according to Example 2 of the present invention.
FIG. 10 is a graph showing changes in expression of endoplasmic reticulum stress response gene during overexpression of miRNA-5100 according to Example 2 of the present invention.
FIG. 11 is a conceptual diagram showing an estimation mechanism of suppression of progression of acute kidney injury by miRNA according to Example 2 of the present invention.

### BEST MODE FOR CARRYING OUT THE INVENTION

### <Embodiment>

It is still unclear whether conventional biomarker candidates for acute kidney injury are truly clinically effective, and no specific treatment method for acute kidney injury has been put into practical use.

Therefore, the present inventors have focused on miRNA, which has been known to be involved in various pathological conditions. It is known that there are about 2000 types of miRNA in mammals including humans, and their base sequences are registered in public databases ("miRBase," or the like).

However, no miRNA useful as a biomarker for acute kidney injury has been known.

Therefore, the present inventors repeated diligent experiments and searched for miRNA that could be a biomarker specific to acute kidney injury.

Specifically, as to be described later in detail in Example 1, the present inventors comprehensively analyzed and identified miRNAs that are changed in the kidney and blood of two types of acute kidney injury model mice by the microarray method and the quantitative real-time reverse transcription PCR (qRT-PCR) method, which is a type of real-time PCR. Further, the identified changes in miRNA expression are compared and examined in human serum samples by qRT-PCR between healthy people and acute kidney injury patients, and miRNAs that specifically change in the serum of acute kidney injury patients is identified and made them usable as a diagnostic method.

Furthermore, it is confirmed in acute kidney injury model mice that overexpression of this miRNA would have an effect when used as a medication, and the present invention has been completed.

### [Biomarker]

More specifically, the acute kidney injury-specific biomarker according to the embodiment of the present invention is miRNA of an acute kidney injury patient.

A miRNA is RNA that is not translated into protein and is a type of functional non-coding RNA. The miRNA is encoded on the eukaryotic genome, undergoes a multi-step production process, and finally becomes a single-stranded RNA of about 20 to 25 bases, which is involved in post-transcriptional expression regulation of genes. By post-transcriptional expression regulation, miRNAs play important roles in a wide range of biological processes such as development, proliferation, differentiation, apoptosis, and metabolism.

Specifically, the acute kidney injury-specific biomarker according to the embodiment of the present invention is miRNA-5100.

Among these, the sequence of mouse mature miRNA-5100 (miRbase No: MI0018008) is shown below:
5'-ucgaaucccagcggugccucu-3' (SEQ ID NO: 1)

The sequence of human mature miRNA-5100 (miRbase No: MI0019116) is shown below:
5'-uucagaucccagcggugccucu-3' (SEQ ID NO: 2)

As the miRNA-5100 of the present embodiment, it is possible to use miRNA having homology in eukaryotes other than mice and humans, specifically, various animals. This animal is not particularly limited, and includes, for example, livestock animal species, wild animals, and the like. Since each miRNA has almost the same base sequence in mammals, it can be searched and used based on homology. The homology is preferably 80% or more, and more preferably 90% or more. Furthermore, it may include sequences that are complementary to these sequences or sequences that are identical to the extent enabling to be hybridized.

In addition, each miRNA of the present embodiment and the functional activity regulator as described later may be provided as a pri-miRNA (primary miRNA, early transcript) or pre-miRNA (precursor miRNA, pre-miRNA). In this case, the miRNA of the present embodiment does not have to be single-stranded, and it may form a double-stranded portion by a stem, a loop structure, or the like.

Further, each miRNA of the present embodiment may be a nucleic acid molecule such as RNA or DNA encoding miRNA, pri-miRNA, pre-miRNA, or the like. The nucleic acid molecule also includes an artificial nucleic acid molecule, for example, a peptide nucleic acid (PNA), a locked nucleic acid (LNA), and the like.

Each miRNA of the present embodiment and the functional activity regulator can be produced by a chemical synthesis method, a recombination method, or the like, which is common to those skilled in the art. In the recombination method, the nucleotide sequence of any of the mature type miRNAs, pri-miRNAs, and pre-miRNAs as described above, or the DNA sequence encoding them, may be included in the appropriate vector. The vector is, for example, an expression vector suitable for expression of nucleic acids in eukaryotes. Nucleic acid molecules included in the vector may target transcription itself of each of miRNA molecule, or a precursor or a primary transcript before being matured into miRNAs. Furthermore, it may include a sequence that modifies the copy or the like, of each miRNA on the genome, a transcriptional regulatory sequence, or the like.

### [Diagnosis method]

The method for diagnosing acute kidney injury according to the embodiment of the present invention is characterized by using the above-mentioned acute kidney injury-specific biomarker.

In acute kidney injury diagnosis method according to the embodiment of the present invention, acute kidney injury is diagnosed primarily by detecting miRNA levels present in the patient's serum or plasma (hereinafter simply referred to as "blood"). That is, the acute kidney injury diagnosis method of the present embodiment can also be used as an examining method of acute kidney injury.

Specifically, in the present embodiment, when the expression of miRNA-5100 is decreased, it is possible to diagnose acute kidney injury.

About the decrease in the expression level of miRNA-5100 measures, for example, the expression level present in the blood of a patient is measured, and whether or not there is a statistically significant difference in the obtained numerical value is tested.

Furthermore, it is possible to diagnose acute kidney injury by combining reduced expression of miRNA-5100 with other diagnoses. These other diagnoses may be, for example, early biomarker candidate molecules for acute kidney injury disclosed in KDIGO. Examples of the molecules may include Neutrophil Gelatinase-Associated Lipocalin (NGAL), Kidney Injury Molecule-1 (KIM-1), and Liver-type Fatty Acid-Binding Protein (L-FABP), which is a fatty acid-binding protein.

To measure the expression level of miRNA-5100 in blood, for example, blood is collected from a patient such as a human, or the like, the supernatant is centrifuged, and total RNA is extracted from serum or plasma excluding blood cells. As a method for extracting total RNA, for example, a guanidine-cesium chloride ultracentrifugation method, an Acid Guanidinium-Phenol-Chloroform (AGPC) method, a column for RNA extraction common to those skilled in the art, or the like, can be used. Then, the expression level of each miRNA of the present embodiment is measured from the extracted total RNA. This measurement can be performed by using a method common to those skilled in the art, such as a Northern blot, a microarray, a Quartz Crystal Microbalance (QCM) sensor measurement method, a real-time PCR method including qRT-PCR, or the like. When the real-time PCR method is used, it is possible to perform expression correction by using an endogenous control miRNA. As the endogenous control in this case, it is preferable for those skilled in the art to appropriately select a sequence that has not been changed between healthy and ill from the sequences previously reported to be appropriate as the endogenous control.

Then, when testing a statistically significant difference from the measured expression level, it is compared with the expression level of miRNA obtained from a sample derived from a healthy person, and for example and is statistically tested whether or not it is 5% significant (p <0.05). In this test, for example, a method such as a T test, an F test, or a chi-square test, or the like, can be appropriately used according to the amount of data, the nature of the data, and the like.

If the test results are statistically significant, the patient can be diagnosed with acute kidney injury. Conversely, if not statistically significant, the patient can be diagnosed as not having acute kidney injury. In this case, from other indicators, or the like, it is also possible to diagnose of a patient with another nephropathy that is not acute kidney injury.

By such a diagnostic method, it is possible to determine with high reliability that the patient has acute kidney injury even in the early stage of onset. This can provide important information for treatment policy decisions.

To summarize the diagnostic method according to the embodiment of the present invention, the diagnostic method of the present embodiment is characterized in that being a method for diagnosing acute kidney injury including: a step of collecting blood from a patient suspected of having acute kidney injury; a step of measuring the expression level of miRNA-5100 in the collected blood; a step of comparing the measured expression level of miRNA-5100 with the expression level (standard amount) of miRNA-5100 in a healthy subject; a step of detecting that the expression level of miRNA-5100 is lower than the standard amount; and a step of determining that a detected patient is at high risk of acute kidney injury.

### [Inspection kit]

The acute kidney injury test kit according to the embodiment of the present invention is characterized by including a reagent for measuring miRNA, which is the above-mentioned acute kidney injury-specific biomarker.

Examples of such a reagent for miRNA measurement include those corresponding to detection methods such as Northern blot, microarray, QCM sensor measurement method, and real-time PCR method. That is, various enzymes such as probes and primers for detecting each miRNA of the present embodiment, buffer solutions, washing solutions, lysates, and the like, are also included. In addition to this, materials, equipment, and the like, for detecting miRNA by the above-mentioned method may be included.

Further, the acute kidney injury test kit of the present embodiment may include a program for determining test results, processing data, and visualizing test results on a computer to analyze diagnostic results, and an apparatus and system provided with the computer, or the like. Such an apparatus, system, or the like, can be developed by using techniques, methods, and the like, which are common to those skilled in the art. Such the apparatus and system, or the like, enable high-throughput examination and facilitates patient diagnosis.

### [Acute kidney injury medication]

The acute kidney injury medication (medical composition) of the present embodiment is characterized by including a composition that increases or decreases the expression level of miRNA.

Specifically, the medical composition of the present embodiment is characterized by including a functional activity regulator of miRNA-5100. The functional activity regulator is a composition having an action of regulating the expression of miRNA.

In the present embodiment, the functional activity enhancer is used as the functional activity regulator for miRNA-5100. The functional activity enhancer includes a miRNA mimic having a base sequence corresponding to miRNA-5100. The miRNA mimic may be a composition including synthesized miRNA. That is, the miRNA mimic enhances the activity of cell function on miRNA-5100 by increasing the concentration of miRNA in the kidney of AKI (overexpression).

The miRNA mimic corresponding to the miRNA-5100 according to the present embodiment can use the following sequences in the case of mouse:
5'-UCGAAUCCCAGCGGUGCCUCU-3' (SEQ ID NO: 3)

As the miRNA mimic corresponding to human miRNA-5100, a similar miRNA corresponding to human miRNA-5100 can be used.

In this case, a miRNA-5100 mimic, or the like, can be used as the dose is, for example, in an amount that is 1.5 to 2.5 times the expression level when miRNA-5100 is administered to a healthy person in the cells of the kidney. Of these, an amount that is about 2.0 times is particularly preferable.

In addition, the medicament for acute kidney injury according to the present embodiment is applied into the cells of a patient in a manner common to those skilled in the art, which is introduced into the desired target cells in vitro or in vivo. Therefore, the medical composition of the present embodiment may be provided including various media common to those skilled in the art.

As the medium, for example, a plasmid or a viral vector may be used. The viral vector may be constructed by using a virus commonly used by those skilled in the art such as adenovirus, adeno-associated virus, retrovirus, or the like.

In addition, the acute kidney injury medication according to the embodiment of the present invention may contain a carrier that is acceptable in any formulation. The carrier may be, for example, a liposome carrier, colloidal gold particles, a polypeptide, a lipopolysaccharide, a polysaccharide, a lipid membrane, or the like. Among these, it is preferable to use a carrier that improves the expression-regulating effect of the functional activity-regulating agent. For example, for miRNA inhibitors and miRNA mimics, it is preferable to use liposomes, especially cationic liposomes.

Further, the pharmaceutically acceptable carrier may include, for example, physiological saline, an isotonic solution containing glucose and other adjuvants, such as D-sorbitol, D-mannose, D-mannitol, sodium chloride, or the like. In addition, it can be administered with suitable solubilizers such as alcohols, specifically ethanol, poly-alcohols such as propylene glycol, polyethylene glycol, nonionic surfactants such as polysorbate 80 (TM), HCO-50, or the like, is possible. In addition, suitable excipients, and the like, may be further included.

In addition, the acute kidney injury medication according to the present embodiment may contain an appropriate pharmaceutically acceptable carrier in order to prepare the pharmaceutically acceptable carrier. The carrier may include biocompatible materials such as silicone, collagen, gelatin, or the like. The carrier may also be provided as an emulsion. Further, for example, one or any combination of pharmaceutical additives such as diluents, fragrances, preservatives, excipients, disintegrants, lubricants, binders, emulsifiers, and plasticizers may be included.

The route of administration of the pharmaceutical composition according to the present invention is not particularly limited, and administration can be performed parenterally or orally. The parenteral administration can be, for example, intravenous, intraarterial, subcutaneous, intradermal, intramuscular, intraperitoneal administration, or direct administration to the kidney, or the like.

The acute kidney injury medication according to an embodiment of the present invention may be formulated in a dosage form suitable for parenteral or oral administration by using a pharmaceutically acceptable carrier well known in the art.

When the acute kidney injury medication according to the embodiment of the present invention is used for the above-mentioned treatment, the administration interval and the dose are appropriately selected and changed according to various conditions such as the condition of the disease and further the condition of the patient.

The single dose and the number of doses of the acute kidney injury medication according to the embodiment of the present invention can be appropriately selected and changed depending on the purpose of administration and further various conditions such as the age and weight of the patient, symptoms and severity of the disease.

The number and duration of administration may be only once or may be administered an extent of once to several times a day for several weeks, the state of the disease may be monitored, and the administration may be repeated or the repeated administration is performed depending on its state.

In addition, the composition of the present invention can be used in combination with another composition, and the like. Further, the composition of the present invention may be administered at the same time as other compositions, or may be administered at intervals, but the order of administration is not particularly limited.

Further, in the embodiment of the present invention, the period for which the disease is improved or alleviated is not particularly limited, but may be temporary improvement or alleviation, or may be improvement or alleviation for a certain period of time.

### [Treatment]

The animal treatment method according to the embodiment of the present invention is an animal treatment method, characterized in that regulating the functional activity of miRNA-5100.

Specifically, the acute kidney injury medication according to the embodiment of the present invention can also be used for animal treatment for treating animals. The animal is not particularly limited and includes a wide range of vertebrates and invertebrates. The vertebrates include fish, amphibians, reptiles, birds, and mammals. Specifically, for example, the mammals are rodents such as mice, rats, ferrets, hamsters, guinea pigs, and rabbits, dogs, cats, sheep, pigs, cows, horses, or non-human transgenic primates, or the like, and may also include humans. In addition to mammals, wild animals include fish, birds including poultry, reptiles, or the like. It also includes a wide range of crustaceans including shrimp and insects, and other invertebrates such as squid.

That is, the acute kidney injury medication according to the embodiment of the present invention can be used not only for human treatment but also methods for various animal treatments, livestock growth promotion, and the like. Therefore, the patient according to the present embodiment includes a human and an animal other than the above-mentioned human.

The treatment method according to the embodiment of the present invention can be summarized. The treatment method according to the present embodiment is characterized in that it is a treatment method for acute kidney injury including: a step of collecting blood from a patient suspected of having acute kidney injury; a step of measuring expression level of miRNA-5100 in the collected blood; a step of comparing the measured expression level of miRNA-5100 with the expression level of miRNA-5100 in a healthy person (standard amount); a step of detecting the expression level of miRNA-5100 being lower than the standard amount; and a step of treating the detected patient with acute kidney injury.

The treatment of this acute kidney injury is characterized by including a treatment that regulates the functional activity of miRNA-5100. In addition, the treatment that regulates the functional activity of the miRNA-5100 is characterized by including administering the acute kidney injury medication as mentioned above.

Specifically, in the present embodiment, regardless of the pathological condition (cause), stage, or the like, as a treatment specific to AKI it is possible to treat AKI by adjusting the functional activity of miRNA-5100. In particular, AKI can be treated by supplemental therapy, or the like, in which miRNA-5100 is overexpressed by administering the above-mentioned acute kidney injury medication. At this time, for example, miRNA-5100 mimic can be administered by DDS (Drug Delivery System) such as the above-mentioned liposome, or the like.

Furthermore, by combining typical treatments performed by those skilled in the art, such as those shown below, it is possible to treat AKI more effectively.

Here, AKI is classified into prerenal, renal, and postrenal etiology according to the pathological condition.

Of these, prerenal AKI is a case in which blood flow to the kidney is reduced, and occurs due to dehydration, decreased blood pressure, and the like. In addition, renal AKI occurs when the kidney itself is impaired. The renal AKI is further subdivided to classification by primary disease, such as vascular acute kidney injury due to cholesterol embolism, renal infarction, or the like, acute glomerulonephritis, lupus nephritis, glomerular disease due to antineutrophil cytoplasmic antibody (ANCA)-related vasculitis, or the like, and acute interstitial nephritis, acute tubular necrosis, renal tubular necrosis caused by drugs, and interstitial acute kidney injury. On the other hand, postrenal AKI is caused by narrowing or obstruction of the urethra. This occurs in bilateral hydronephrosis, or the like.

In the treatment of AKI by typical pathology (cause), it is performed that fluid replacement for prerenal AKI, treatment of the primary disease for renal AKI, and treatment such as urethral stenosis and relief of obstruction for postrenal AKI.

In addition, according to the KDIGO practice guidelines for AKI, stage-specific treatment of typical AKI, in "high risk" conditions, therapies such as discontinuing nephrotoxic substances as much as possible, guaranteeing fluid volume and reflux pressure, considering functional hemodynamic monitoring, monitoring serum creatine levels and urine output, prevent hyperglycemia, considering alternatives that do not use contrast media, and the like, are performed. Moreover, at Level 1, in addition to these, non-invasive evaluation is performed, and invasive evaluation is also considered. At level 2, further in addition to these, therapies such as adjusting the drug dose according to renal function, considering renal replacement therapy, and considering admission to the Intensive Care Unit (ICU) are performed. At level 3, further avoid subclavian catheters if possible.

In addition, the acute kidney injury medication according to the embodiment of the present invention can also be a therapeutic target for a part of the body of an animal, or an organ or tissue, or the like, that is removed or excreted from the animal. Furthermore, this treatment is a treatment in a broad sense, and it can be applied to a bioreactor, a culture in a model animal, a culture of a human transplant-like cultured organ, or the like.

As configured in this way, the following effects can be obtained.

AKI is diagnosed in the KDIGO classification based on an increase in serum creatinine level, a decrease in urine volume, and the like. However, the criterion often missed the timing of treatment interventions. In addition, it was not clear whether the KDIGO diagnostic criteria could be used to predict renal prognosis.

Therefore, in recent years, early biomarker candidate molecules for acute kidney injury such as urinary NGAL and L-FABP have also been reported.

Specifically, NGAL, which is a conventional early biomarker candidate molecule for acute kidney injury, measures Neutrophil Gelatinase-Associated Lipocalin in urine and can be used as a diagnostic aid for acute kidney injury (AKI). The area under the Receiver Operating Characteristic (ROC) curve of NGAL was 0.50 to 0.98, and in 75% (12/16) studies, the diagnostic accuracy was 0.70 or higher, which was moderate or higher (see AKI Clinical Guidelines 2016).

On the other hand, L-FABP is a 14 kd protein localized in the cytoplasm of the human proximal tubule, and it is used for early diagnosis of renal tubular dysfunction because of being excreted in urine due to ischemia to the renal tubules and oxidative stress. The area under the ROC curve of L-FABP was 0.70 to 0.95, and the diagnostic accuracy was 0.70 or higher and moderate or higher in all studies (as refer to Clinical Practice Guideline for Acute Kidney Injury 2016).

However, it remains unclear whether these biomarker candidates are truly clinically effective. This was because there were multiple measurement methods, they were not standardized, and the timing of measurement was not fixed. Furthermore, in L-FABP, the cutoff value was not determined. That is, it was not known whether these conventional biomarker diagnoses were truly useful.

Further, in the first place, the miRNA as described in Patent Document 1 was not analyzed for miRNA specific to acute kidney injury.

On the other hand, the acute kidney injury-specific biomarker according to the embodiment of the present invention is miRNA present in blood, and by using miRNA-5100, it enables to use for the diagnosis of acute kidney injury.

That is, the biomarker of the present embodiment can accurately diagnose acute kidney injury, which is a renal disease with a poor prognosis, at an early stage, and it can be expected to have a therapeutic effect.

In addition, in the method for diagnosing acute kidney injury according to the embodiment of the present invention, it is possible to diagnose acute kidney injury among nephropathy at an early stage and non-invasively.

Furthermore, as shown in Example 2 below, the biomarker of the embodiment can diagnose acute kidney injury at an earlier stage (stage 1) than conventional NGAL and L-FABP

In addition to this, NGAL and L-FABP, which are conventional early biomarker candidate molecules for acute kidney injury, may not be able to be measured in patients who do not have urine because the sample at the time of measurement is urine.

On the other hand, since the biomarker of the present embodiment can be measured by using blood (serum) as a sample, it can be reliably measured even in a patient who does not have urine.

Meanwhile, a specific treatment method for acute kidney injury has not been put into practical use at all. That is, there was no specific therapeutic agent for the treatment of acute kidney injury.

Specifically, conventional guidelines for acute kidney injury have covered low-dose atrial natriuretic peptide administration, loop diuretic administration, low-dose dopamine administration, and blood purification therapy as drugs and dialysis therapy. However, there was insufficient evidence of administration of low-dose atrial natriuretic peptides in the prevention and treatment of acute kidney injury. In addition, loop diuretic administration is not recommended for the prevention of acute kidney injury, and it was proposed that loop diuretics may not be administered as a treatment for acute kidney injury except for use to correct fluid excess. Further, administration of low-dose dopamine was not recommended for the prevention and treatment of acute kidney injury. In addition, there is little evidence that early initiation of blood purification therapy improves the prognosis for acute kidney injury, and the timing of initiation has been determined with broad consideration of clinical symptoms and pathophysiology.

Thus, in acute kidney injury, there are no established therapeutic agents or treatments, and they are limited to symptomatic treatments such as avoiding infusions and causative agents.

On the other hand, by using a functional activity regulator of miRNA-5100, a novel acute kidney injury medication can be provided, and early treatment can be performed in combination with the above-mentioned diagnosis.

In particular, by replacement therapy or the like in which miRNA-5100 is overexpressed by administering miRNA-5100 mimic with DDS, or the like, apoptosis of ureteral cells and the like can be suppressed, and deterioration of renal function due to the onset and progression of AKI can be prevented and suppressed.

Specifically, necrosis of renal tubules and filling of protein columns in the renal tubules can be suppressed. Furthermore, it can be expected that the onset and progression of acute kidney injury can be suppressed by prophylactic administration at the time of occurrence or predicted occurrence of acute kidney injury.

In addition, in the above-described embodiment, an example of obtaining miRNA from blood as a test method for acute kidney injury has been described.

However, for example, miRNAs are stably present in urine other than in blood. Therefore, it can be configured to detect AKI based on the amount of miRNA obtained from a tissue, body fluid, urine, or the like, other than blood.

Further, the diagnostic method of the present embodiment can also be used as a preliminary diagnostic method for early diagnosis. That is, in the case of acute kidney injury, as described above, it may be too late for the conventional diagnosis. Therefore, by diagnosing acute kidney injury at an early stage, it is possible to enable accurate early treatment and improve the therapeutic effect.

In addition, as a functional activity regulator of miRNA-5100, which is an acute kidney injury medication, it is also possible to use a composition other than miRNA mimic.

Furthermore, a gene, a gene product, an agonist / an antagonist, or a composition through actions on another pathway, which is a target of expression regulation of miRNA-5100, can also be used as a functional activity regulator.

Furthermore, as a functional activity regulator of miRNA-5100 as an acute kidney injury medication, other miRNA transcriptional regulators may be used, and it is also possible to regulate expression with various vectors, perform gene therapy with CRISPR / Cas, or the like.

In addition, it is also possible to perform gene therapy through the action on a gene or a pathway, which is the target of expression regulation by miRNA-5100.

In addition, the drug according to the embodiment of the present invention can be used in combination with another composition, or the like. The composition of the present invention may be administered, sprayed, applied, or the like, at the same time as the other composition.

In addition, miRNA-5100 functional activity regulator can also be used for a non-pharmaceutical application. Further, as functional activity regulator, an activity inhibitor such as a miRNA inhibitor, or the like, may be used. This makes it possible to use it for experiments and models, or the like, of the mechanism of action in acute kidney injury in animals.

The miRNA inhibitor includes, for example, various compositions containing nucleic acid molecules such as antisense, siRNA, ribozyme, or the like, which inhibits the production of mature miRNAs or suppress expression by cleaving pri-miRNA and pre-miRNA, or the like, can be used.

### [Example 1]

Hereinafter, the acute kidney injury-specific biomarker according to the embodiment of the present invention is described more specifically as an example based on a specific experiment. However, this embodiment is merely an example, and the present invention is not limited thereto.

### [Materials and methods]

### (Microarray analysis)

Analysis of miRNA expression was performed outsourced to Hokkaido System Science, Inc. (Hokkaido, Japan) by using microRNA Complete Labeling Reagent and Hyb kit (Agilent Technologies, CA, USA). 100 ng of total RNA was dephosphorylated with calf intestinal phosphatase at 37 degree-C for 30 minutes and denatured with 100% dimethyl sulfoxide at 100 degree-C for 7 minutes. The sample was then cooled on ice for 2 minutes. Then, samples were then incubated with T4 ligase at 16 degree-C for 2 hours and labeled with pCp-Cy3. Then, the pCp-Cy labeled sample was hybridized on an 8x15K format Agilent mouse microRNA array. Then, the plates were then washed and scanned by using an Agilent Technologies Microarray scanner with a resolution of 3 micro-m. The data were analyzed using Agilent Feature Extraction software version 10.7.3.1.

### (Microarray data processing and statistical analysis)

The Agilent result data was imported into GeneSpring GX (manufactured by Agilent Technologies) and normalized to the 90th percentile per chip. Differences between different groups were analyzed by using One-way Analysis Of Variance (ANOVA). When statistical significance was detected by ANOVA, Tukey's test was performed as a post-hook test to compare the mean of two different groups. P <0.05 was determined to be significantly different.

### (qRT-PCR)

### (Mouse kidney)

Mouse kidneys were homogenized by using a glass homogenizer and a filter column shredder (QIA shredder, Qiagen, Valencia, CA, USA). Then, by using a miRNeasy mini kit (Qiagen), total RNA containing miRNA was isolated from the homogenized kidney sample. Next, 1 micro-g of isolated total RNA was performed reverse transcription by using the miScript II RT kit (Qiagen). Next, real-time RT-PCR (qRT-PCR) was performed using the miScript SYBR green PCR kit (Qiagen). The conditions for qRT-PCR by using the QuantStudio 12K Flex Real-Time PCR system were preincubation at 95 degree-C for 15 minutes is performed, then, 40 cycles of (1) denaturation at 94 degree-C for 15 seconds, (2) 30 seconds at 55 degree-C annealing, and (3) extension at 70 degree-C for 30 seconds were performed, and the results were analyzed by 2-delta delta CT method by using RNU6-2 (manufactured by QUIAGEN) as an endogenous control.

### (Human serum)

For miRNA level analysis of human serum, total RNA was isolated from 400 micro-l serum by using a NucleoSpin miRNA Plasma column (Machery-Nagel, PA, USA). The isolated total RNA was then performed reverse transcription by using the miScript II RT kit (Qiagen). Then, qRT-PCR was performed by using the miScript SYBR green PCR kit (Qiagen). The conditions for qRT-PCR by using the Quant Studio-12K Flex Real-Time PCR system were preincubation at 95 degree-C for 15 minutes is performed, then, 40 cycles of (1) denaturation at 94 degree-C for 15 seconds, (2) 30 seconds at 55 degree-C annealing, and (3) extension at 70 degree-C for 30 seconds were performed, and the results were analyzed by 2-delta delta CT method by using miRNA-423-3p as the endogenous control.

The following primers were used.

The primer used for mouse miRNA-5100 PCR (manufactured by QIAGEN):
5'-UCGAAUCCCAGCGGUGCCUCU-3' (SEQ ID NO: 4)

The primer used for human miRNA-5100 PCR (manufactured by QIAGEN):
5'-UUCAGAUCCCAGCGGUGCCUCU-3' (SEQ ID NO: 5)

### (Acute kidney injury model mouse)

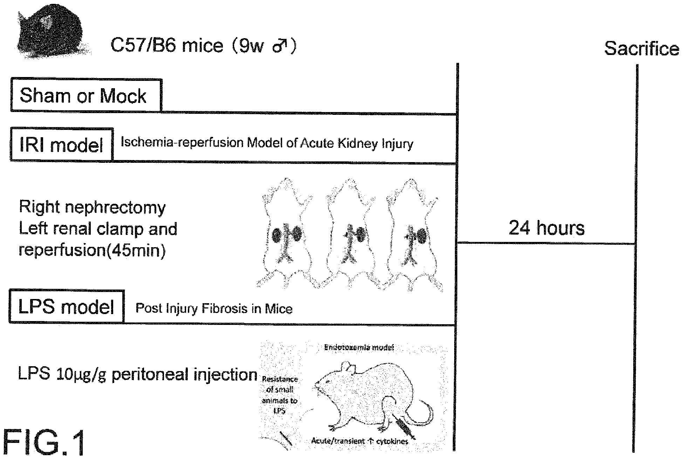

The acute kidney injury model mouse used in the test is described with reference to FIG. 1. In Example 1, an ischemia-reperfusion model mouse (IRI Model) and a lipopolysaccharide (LPS) -administered model mouse (LPS Model) were prepared.

In the ischemia-reperfusion model mouse, after excision of the right kidney of C57 / B6 mice (9 weeks old, male), the left renal artery was clamped for 45 minutes to block renal blood flow to induce acute renal failure, and they were dissected after 24 hours.

LPS-administered model mice were administered with a single dose of Lipopolysaccharide (LPS, 10 micro-g / g) PBS aqueous solution to C57 / B6 mice (9 weeks old, male) to induce acute renal failure, and they were dissected after 24 hours.

These underlying mice and other control mice were purchased from the Animal Breeding Institute.

### (Overexpression of microRNA-5100)

### (miRNA-5100 mimic and control miRNA)

The miRNA-5100 mimic for overexpression in mice and the control miRNA were purchased from Gene Design Inc.

The sequence of miRNA-5100 mimic (manufactured by Gene Design Inc.) is the same as that of SEQ ID NO: 3 as described above.

The control miRNA (Control-miRNA) is a synthetic sequence that is not homologous to the miRNA registered in miRBase and is a negative control.

### (Preparation of miRNA-5100 mimic-PEI-NP)

As a non-viral carrier for administering miRNA-5100, a nano-sized liposome, Linear polyethylenimine-based nanoparticle (PEI-NP), was used. PEI-NP is preferably used for administration of miRNA because of its excellent biocompatibility, stability and transfection ability. In Example 1, in vivo-jetPEI (registered trademark, manufactured by Polyplus-transfection) was used as PEI-NP.

Specifically, miRNA-5100 mimic was dissolved in a 5% glucose solution at a concentration of 50 micro-M. PEI-NP was dissolved in a 5% glucose solution. The miRNA mimics and PEI-NPs were mixed and incubated at room temperature for 15 minutes to prepare miRNA-5100 mimic-PEI-NPs. This causes the condensed miRNA-5100 mimic to be encapsulated in liposomes.

By using the same method, control-miRNA-PEI-NPs were prepared by mixing PEI-NPP with control miRNA.

### [result]

### (Microarray analysis and qRT-PCR results)

In Example 1, we analyzed miRNA that changes with acute kidney injury and examined its potential as a biomarker. Therefore, in order to exclude the effects of experimental factors such as surgical procedures and drug administration itself, two established types of acute kidney injury model mice with different mechanisms were used, and miRNAs that were changed in the kidney of AKI were comprehensively analyzed by the microarray method.

Of these, the ischemia-reperfusion model mouse is an acute kidney injury model mouse due to ischemia after surgery, or the like. On the other hand, the LPS-administered model mouse is an acute kidney injury model mouse in septic poisoning.

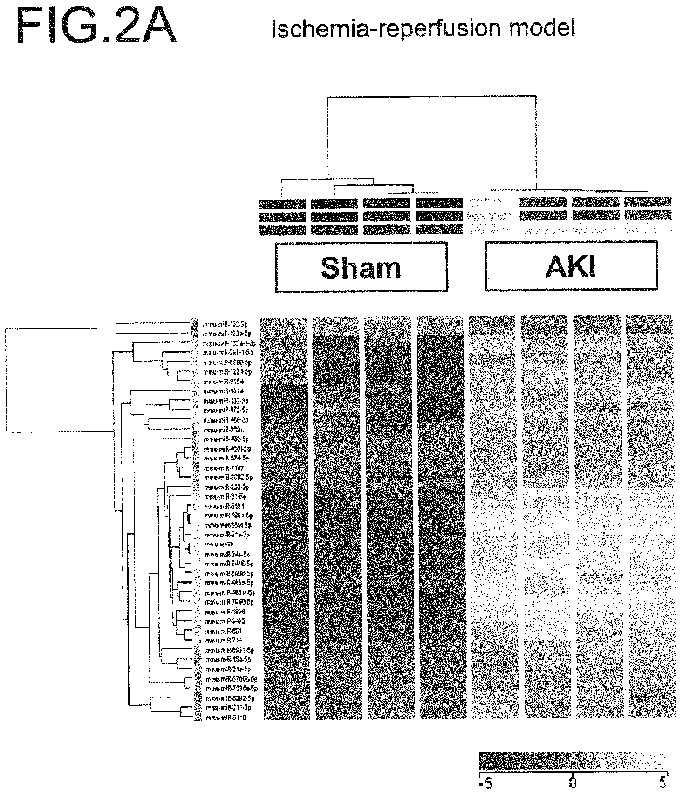

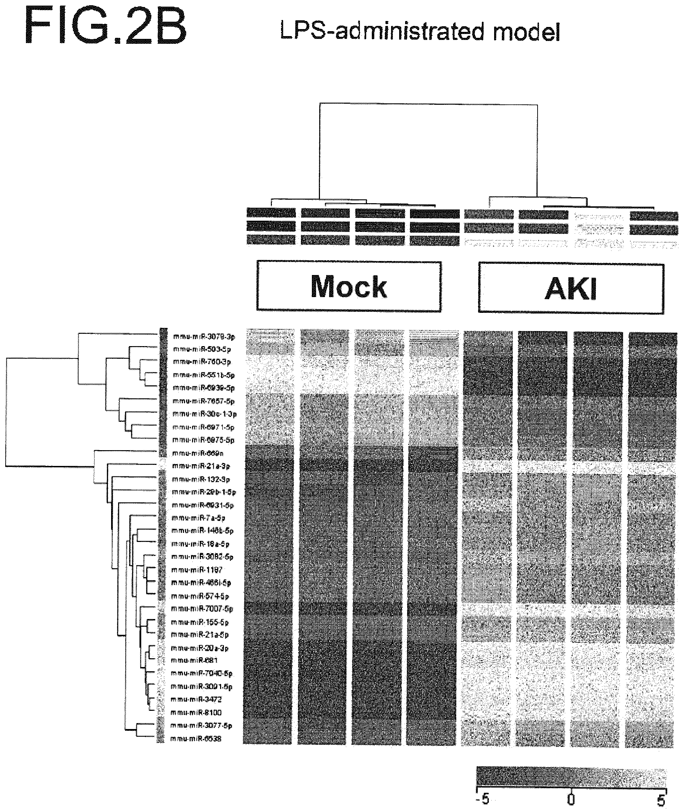

Firstly, microRNAs that commonly change in acute kidney injury were comprehensively analyzed by the microarray method. FIG. 2A and 2B show the results of microarray analysis. FIG. 2A shows the results in the ischemia-reperfusion model mouse. FIG. 2B shows the results in LPS-administered model mice.

Microarray analysis of 1881 types of miRNA were performed in the kidneys of each acute kidney injury mouse (4 mice) and normal mouse (control mouse) (4 mice), respectively.

As a result, among the 1881 types of miRNA, 18 types of miRNAs that are 1.2-fold or more elevated and 19 types of miRNAs that are 1.2 times or more lower in the both kidneys of ischemia-reperfusion acute kidney injury mice and LPS-administered acute kidney injury mice, which is a total of 37 types, were selected.

### (QRT-PCR results in humans)

Regarding miRNAs that specifically change in mouse serum, miRNAs that change in humans with serum compared with healthy people were searched for, and miRNAs that serve as biomarkers for acute kidney injury were identified.

About each of 37 types of miRNAs where the expression was significantly changed in the kidneys of acute kidney injury model mice, miRNA was extracted from the sera of acute kidney injury patients (31 cases) (diagnosed by KDIGO classification) and healthy people (23 cases) by using NucleoSpin (registered trademark) miRNA Plasma kit, cDNA is generated by using miScript II RT Kit (registered trademark), the expression of miRNA was compared and examined by qRT-PCR.

Thereby, miRNA-5100, which is a miRNA whose expression is specifically reduced only in the serum of patients with acute kidney injury, was identified.

### (Expression analysis of miRNA-5100)

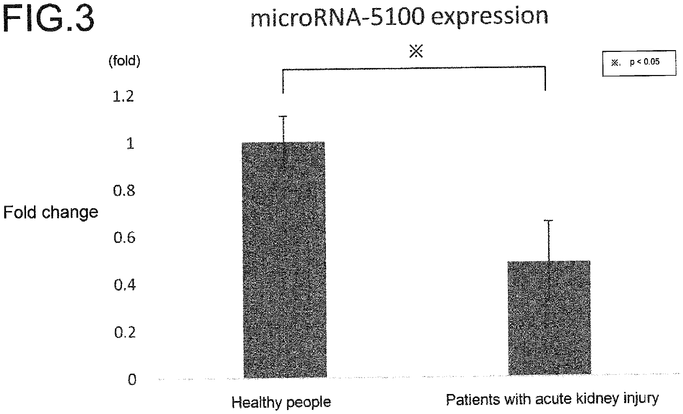

Next, changes in the expression level of miRNA-5100 is described with reference to FIG. 3.

FIG. 3 is a graph showing the difference in the expression level by qRT-PCR by normalizing a healthy person as 1. The expression level of miRNA-5100 was significantly reduced at P < 0.05.

Table 1 below shows the mean and standard deviation of the analysis results.

**[Table 1]**

| | Healthy person control (n=23) | Acute kidney injury AKI (n=31) |
|---|---|---|
| average | 1 | 0.484469625 |
| SE | 0.11 | 0.17 |

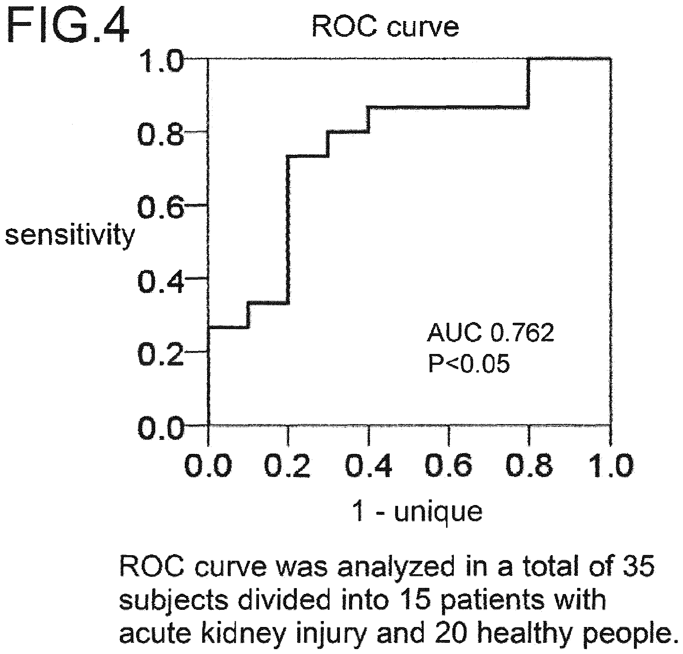

FIG. 4 is a graph showing the accuracy evaluation (credibility) of miRNA-S100 by the ROC curve. Here, an analysis was performed on a total of 35 subjects divided into 15 patients with acute kidney injury and 20 healthy people. The AUC was 0.762, which was a specific marker with P < 0.05.

The test results for the area product below this curve are shown in Table 2 below.

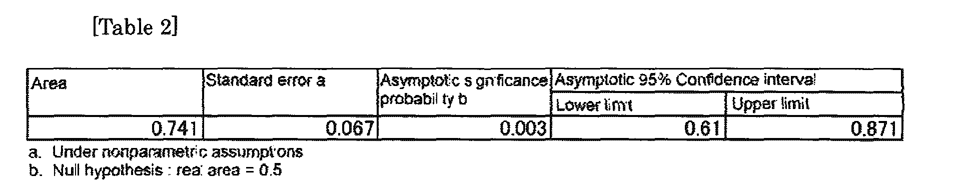

(Examination of miRNA-5100 as an acute kidney injury medication)

Next, the effect of miRNA-5100 as a medication on acute kidney injury was examined in acute kidney injury model mice.

Specifically, miRNA-5100 mimics are administered by using PEI-NP to reach the kidneys, the effect was evaluated by measuring the expression level of miRNA-5100 in the kidney, the renal weight, and the amount of molecules that increased reflecting renal damage.

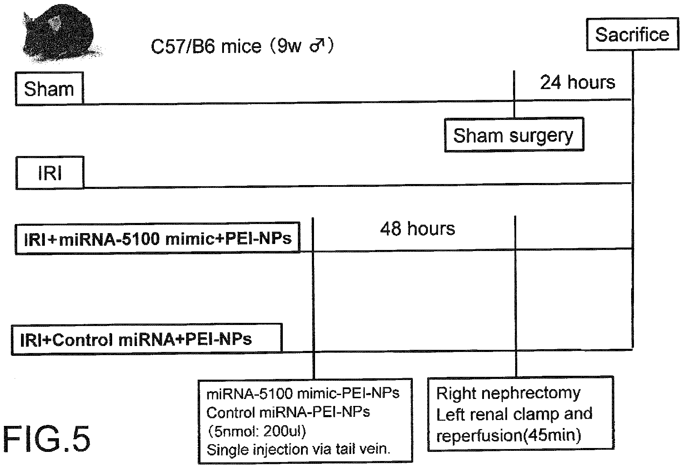

According to FIG. 5, in the actual administration, a total of 200 micro-L of miRNA-5100-PEI-NP (miRNA: 5 nmol, N / P ratio = 6) was intravenously injected from the tail vein into each mouse according to the protocol of Gene Design Inc.

Here, miRNA-5100 mimic-PEI-NPs administered to ischemia-reperfusion model mice were used as the administration group. As controls for this, a group of mouse which an operation of ischemia-reperfusion model is not performed and administration of PEI-NP was not performed (pseudo-surgery, Sham), a group of ischemia-reperfusion model mouse without administration of PEI-NP (IRI), and a group in which control-miRNA-PEI-NPs were administered to ischemia-reperfusion model mice (control miRNA-administered group) were used.

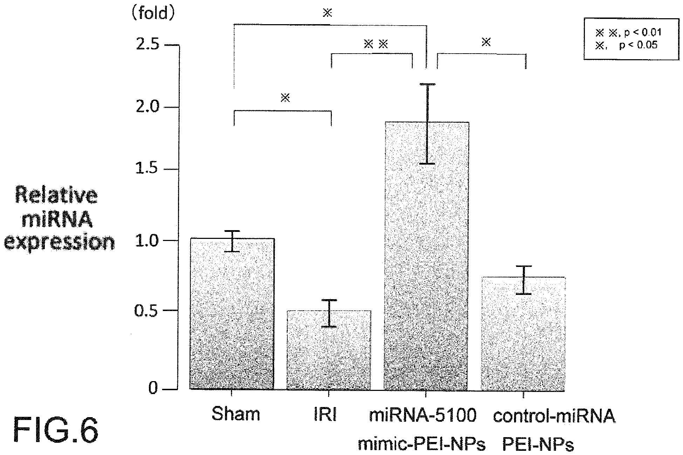

FIG. 6 is a graph showing the results of the expression level of miRNA-5100 in the kidney. In each graph, the relative expression level of miRNA-5100 with the sham value set to 1 after the t-test is shown. The "*" indicates that p <0.05, and the "**" indicates that p < 0.01 as significant.

As a result, in the administration group (miRNA-5100 mimic-PEI-NPs), the relative expression level of miRNA-5100 in the kidney was increased statistically significantly for each control.

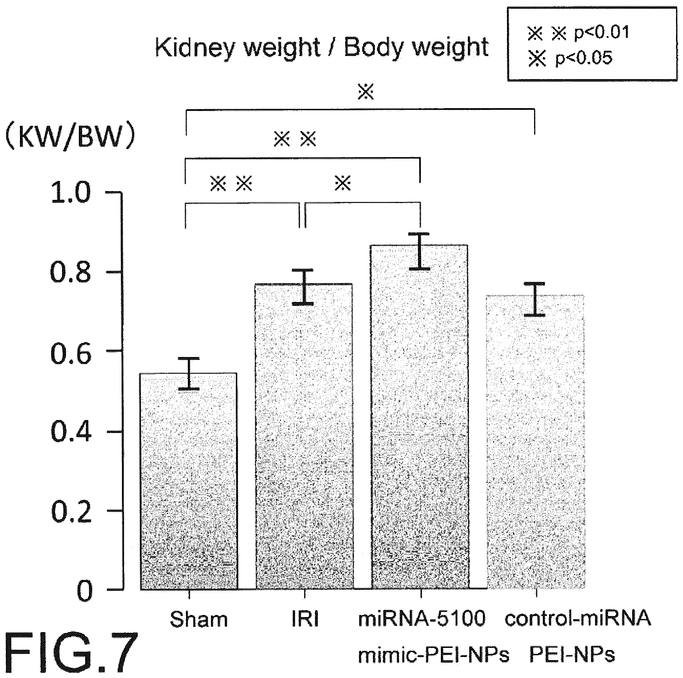

FIG. 7 is a graph showing bilateral kidney weight (mg) / body weight (g) in each group. In each graph, the value of sham after the t-test is set to 1.

As a result, the miRNA-5100-administered group (miRNA-5100 mimic-PEI-NPs) showed the most renal swelling. In preliminary experiments, it is speculated that in acute kidney injury, more renal weight may reduce damage to the kidney.

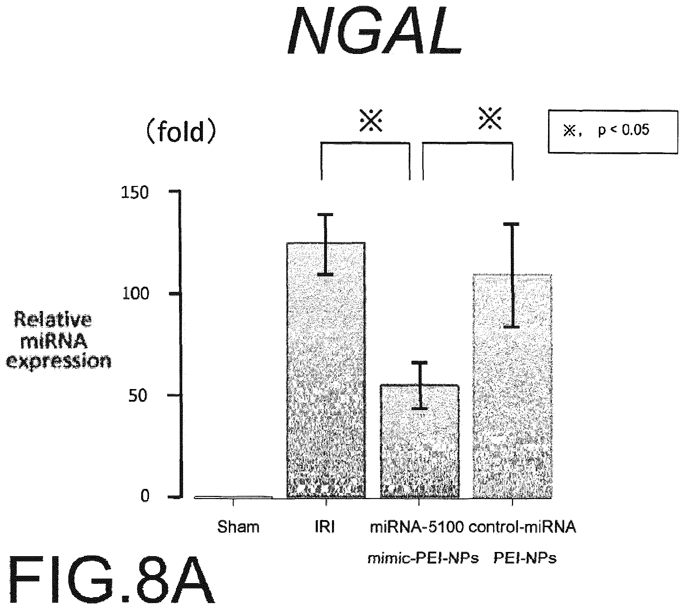

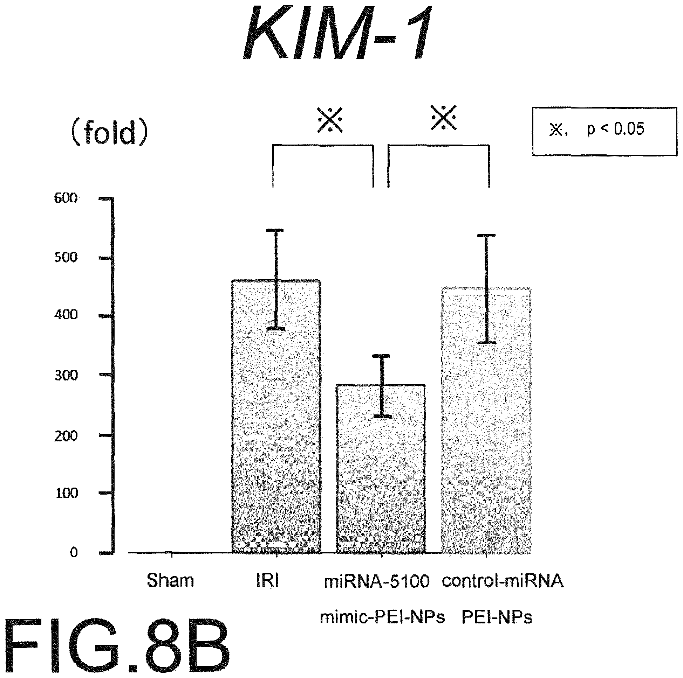

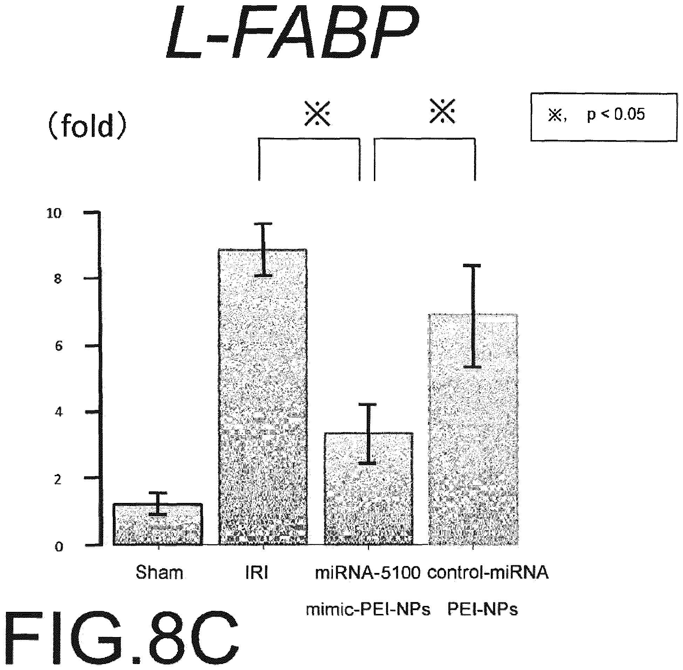

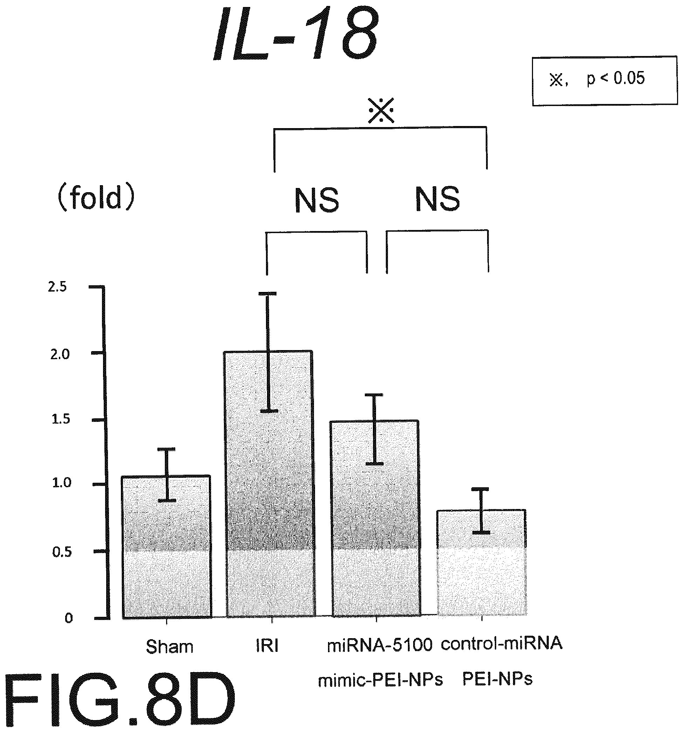

Next, the measurement results of the amount of molecules that increase reflecting renal damage by administration of miRNA-5100 mimic is described with reference to FIGS. 8A to 8D. As these molecules, NGAL, KIM-1 (Kidney injury molecule-1), L-FABP, IL-18 (Interleukin 18), which are biomarker "candidate" molecules introduced in the KDIGO Clinical Practice Guideline Kidney Injury, were used. FIG. 8A is a graph showing the relative expression level of NGAL for each group. FIG. 8B is a graph showing the relative expression level of KIM-1 for each group. FIG. 8C is a graph showing the relative expression level of L-FABP for each group. FIG. 8D is a graph showing the relative expression level of IL-18 for each group. Each figure shows the relative expression level with the value of sham set to 1 after the t-test.

About NGAL, KIM-1, and L-FABP, expression was significantly reduced at p < 0.05 in the miRNA-5100-administered group (miRNA-5100 mimic-PEI-NPs) with respect to the ischemia-reperfusion model mouse group (IRI) and the control miRNA-administered group (control-miRNA-PEI-NPs). That is, overexpression of miRNA-5100 in the kidney significantly suppressed molecules that were elevated in acute kidney injury reflecting renal injury.

Thus, the effect was shown that miRNA-5100 may be a therapeutic agent for AKI.

In addition, regarding IL-18, the miRNA-5100-administered group was not significant in the statistical test with respect to the ischemia-reperfusion model mouse group and the control miRNA-administered group. However, in the test for the ischemia-reperfusion model mouse group and the control miRNA-administered group, p < 0.05. Therefore, it is suggested that the expression level of IL-18 may be changed by some mechanism by administration of miRNA after the treatment of the ischemia-reperfusion model mouse. That is, it is speculated that IL-18 may have low specificity as a biomarker in acute kidney injury due to ischemia such as after surgery.

### [Example 2]

### [Human diagnosis results]

### (Comparison of miRNA-5100 with conventional AKI markers)

For actual patients with acute kidney injury (14 cases), the conventional AKI markers urinary NGAL value and urinary L-FAB value were compared with the diagnosis based on the decreased expression of miRNA-5100.

The diagnosis due to the decreased expression of miRNA-5100 is the same as in Example 1 described above.

After collecting urine from the patient, urinary NGAL was centrifuged at 400G and more than 5 minutes, the supernatant was collected, submitted to SRL Co., Ltd., and measured by the CLIA method.

For L-FABP in urine, the collected urine was stored in a refrigerator, and it was measured by the CLEIA method at SRL Co., Ltd.

The results are shown in Table 3 below.

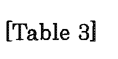

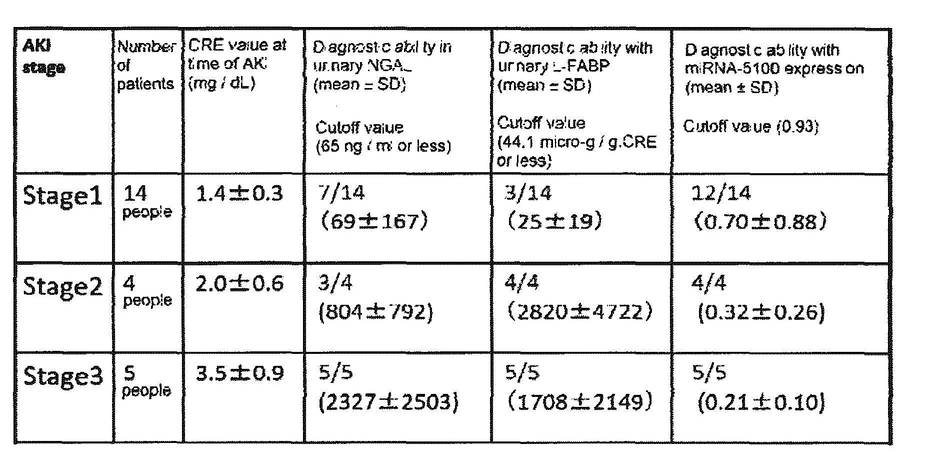

In Table 3 above, the three patients who could not measure NGAL and L-FABP due to anuria were excluded and compared.

As a result, decreased expression of miRNA-5100 was also observed in early stage (stage 1) acute kidney injury in which NGAL and L-FABP did not change. Therefore, miRNA-5100 is considered to be superior to NGAL and L-FABP as a biomarker for acute kidney injury.

### [Estimated mechanism of suppression for progressing acute kidney injury]

In above mentioned Example 1, it was shown that NGAL and KIM-1, which are markers of renal tubular stromal cell damage, were suppressed during overexpression by administration of miRNA-5100 mimic. For these, it was considered that it has a possibility involved in the mRNA of the ATF-6 pathway and the PERK pathway among the three pathways, which are ATF-6 pathway, PERK pathway, and IRE-1 pathway, during endoplasmic reticulum stress (ER stress). Therefore, in the same manner as in Example 1 described above, the expression of genes in the ATF-6 pathway and the PERK pathway after administration of miRNA-5100 mimic was separately measured by qRT-PCR.

As a result, the expression of ATF-6, BID, IP3R, and P53 was significantly changed. Of these, ATF-6 (Activating Transcription Factor 6) is a membrane-bound transcription factor belonging to the CREB / ATF family. It activates the expression of Unfolded Protein Response (UPR) protein. BID is one of the apoptosis-promoting Bcl-2 family proteins. IP3R is an inositol trisphosphate receptor that is integrated into the endoplasmic reticulum membrane inside the cell. P53 is a tumor suppressor gene associated with apoptosis.

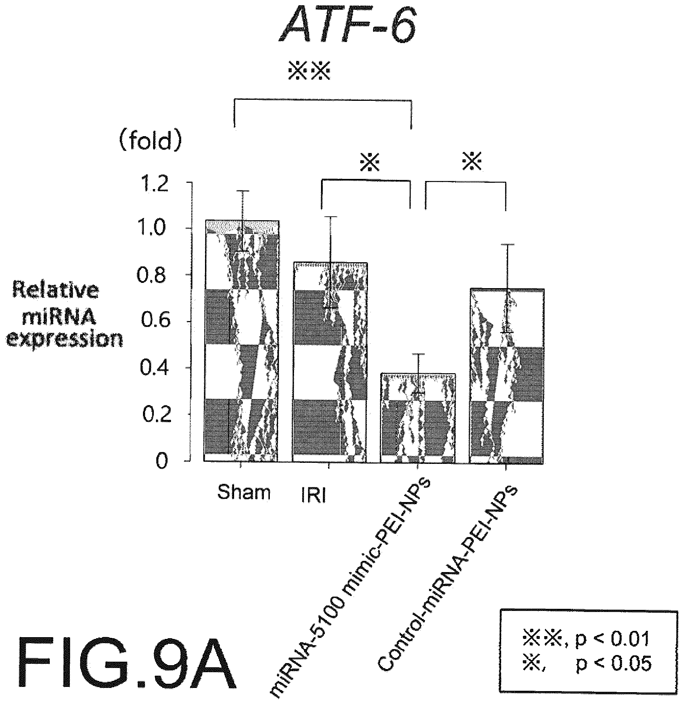

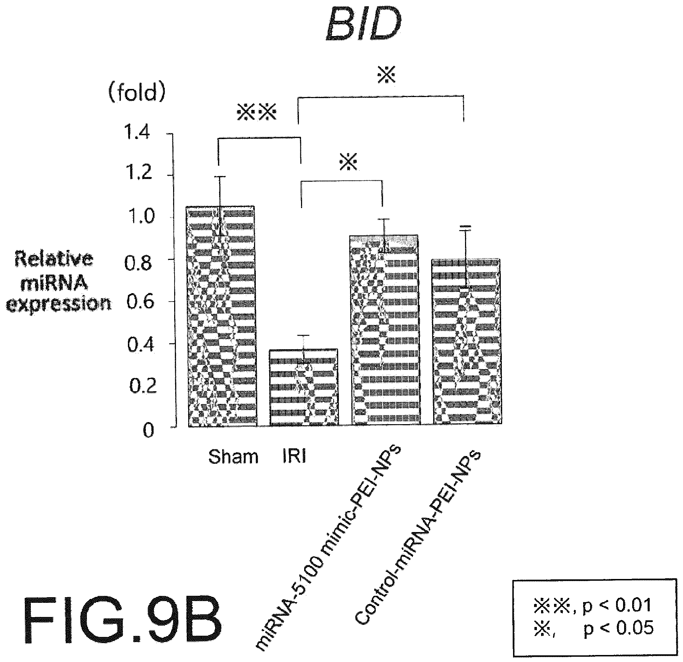

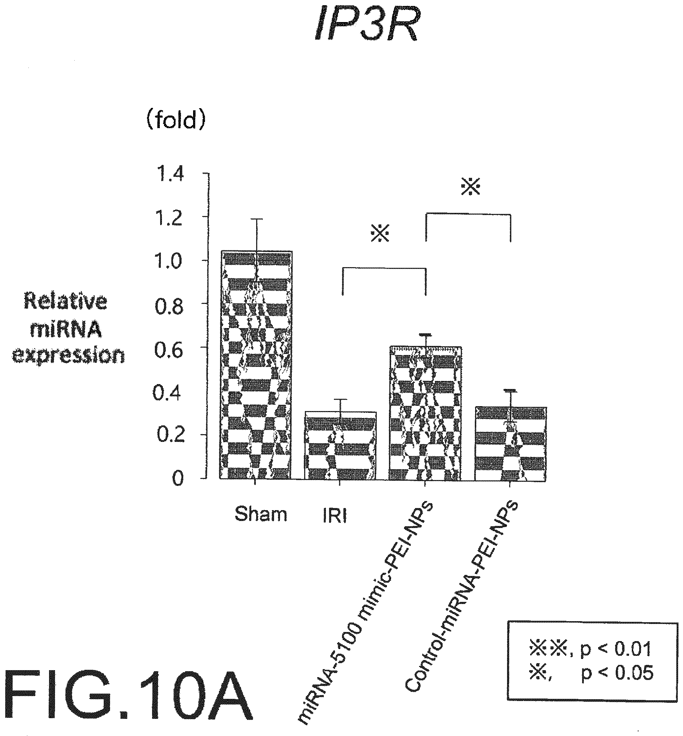

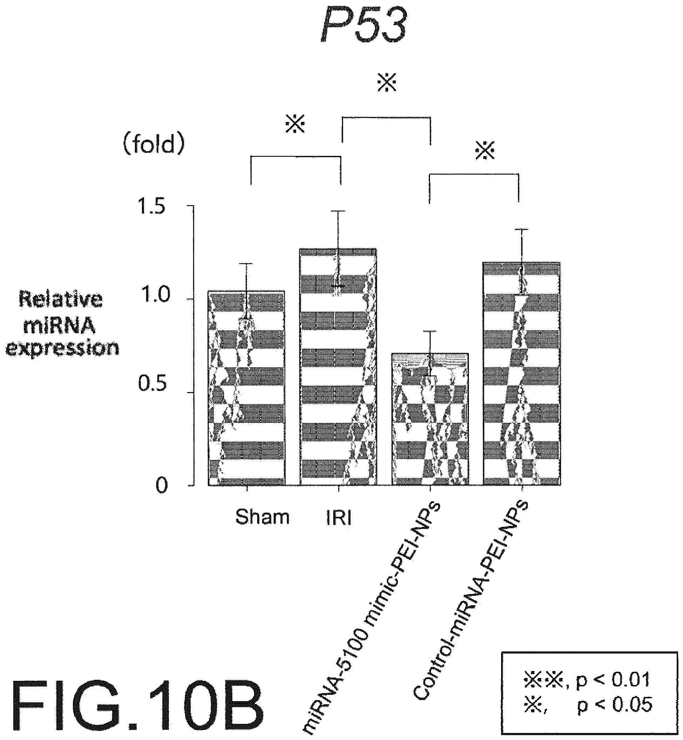

FIGS. 9 and 10 show graphs indicating the relative expression levels of these molecules for each group. FIG. 9A is a graph showing the relative expression level of ATF-6 for each group. FIG. 9B is a graph showing the relative expression level of BID for each group. Each figure shows the relative expression level with the value of sham set to 1 after the t-test.

The expression of both ATF-6 and BID was decreased by administration of miRNA-5100 mimic. These genes are associated with the ATF-6 pathway of endoplasmic reticulum stress response. That is, it is considered that administration of miRNA-5100 mimic can suppress apoptosis by reducing the expression of these genes, by suppressing BiP / GRP78 and GRP94 downstream of the pathway, and also by suppressing XBP-1 and CHOP,

FIG. 10A is a graph showing the relative expression level of IP3R for each group. FIG. 10B is a graph showing the relative expression level of P53 for each group. These figures also show the relative expression level with the sham value set to 1 after the t-test.

The expression of IP3R was significantly increased by administration of miRNA-5100 mimic. On the other hand, the expression of P53 was decreased by administration of miRNA-5100 mimic. These genes are associated with the PERK pathway of endoplasmic reticulum stress response. Specifically, when the expression of IPR is increased, the decrease in Ca influx into the endoplasmic reticulum is suppressed, and apoptosis is suppressed. Furthermore, when P53 expression is reduced, apoptosis is suppressed.

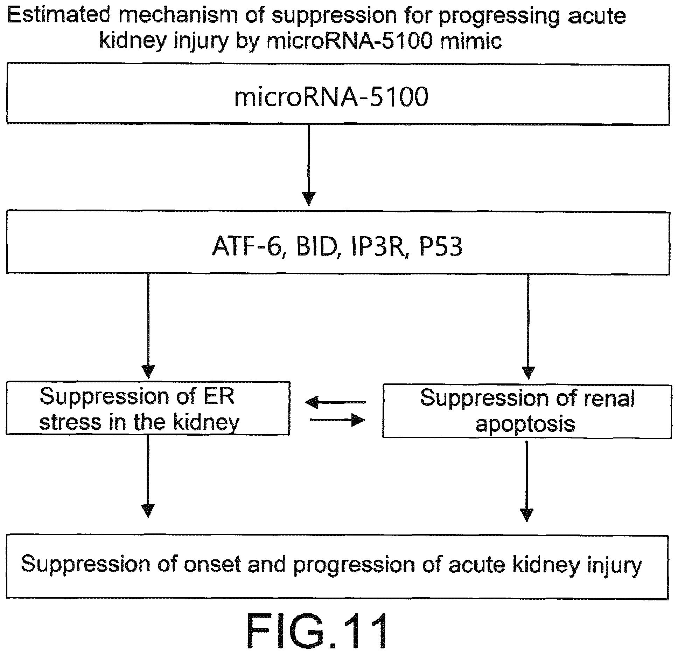

FIG. 11 shows a summary of the estimated mechanism by which the progression of acute kidney injury was suppressed by these genes.

That is, it is considered that administration of miRNA-5100 mimic changes the expression of ATF-6, BID, IP3R, P53, suppresses renal endoplasmic reticulum stress, suppresses renal apoptosis, and, as a result, suppresses the onset and progression of acute kidney injury.

Needless to say, the configuration and operation of the above-described embodiment are examples and can be appropriately modified and executed without departing from the aim of the present invention.

### [Industrial applicability]

According to the present invention, by using miRNA present in blood as a biomarker specific to acute kidney injury, early diagnosis of acute kidney injury and provision of therapeutic drugs can be expected, and it can be industrially used.

## Claims

1. Acute kidney injury-specific biomarker, **characterized in that** an acute kidney injury-specific biomarker is miRNA-5100.

2. An acute kidney injury diagnosis method **characterized in that** includes the step of:
using the acute kidney injury-specific biomarker according to claim 1.

3. The acute kidney injury diagnosis method according to claim 2, further **characterized in that** includes the step of:
diagnosing as acute kidney injury when an expression of the miRNA-5100 is decreased.

4. An acute kidney injury test kit **characterized in that** includes:
a reagent for measuring miRNA-5100.

5. An animal treatment method for an animal other than a human **characterized in that** includes the step of:
regulating functional activity of miRNA-5100.

6. An acute kidney injury medication **characterized in that** includes:
a functional activity regulator for miRNA-5100.

7. The acute kidney injury medication according to claim 6, **characterized in that**
the functional activity regulator includes functional activity enhancer of the miRNA-5100.

8. The acute kidney injury medication according to claim 7, **characterized in that** the functional activity enhancer is a miRNA mimic corresponding to the miRNA-5100.
